# FASCICULE DE BREVET EUROPEEN

(11) **EP 2 413 887 B1**
(45) Date de publication et mention de la délivrance du brevet: **08.10.2014**
(21) Numéro de dépôt: 10718635.5
(22) Date de dépôt: 30.03.2010
(51) Int. Cl.: A61K 8/33, A61Q 3/00, A61Q 3/02

(54) **COMPOSITION COSMETIQUE DURCISSANTE POUR LES ONGLES, UTILISATION D'ALDEHYDES POUR DURCIR ET RENFORCER LES ONGLES ET LEUR PROCEDE D'APPLICATION**
KOSMETISCHE ZUSAMMENSETZUNG ZUM HÄRTEN DER NÄGEL, VERWENDUNG VON ALDEHYDEN ZUR HÄRTUNG UND STÄRKUNG DER NÄGEL UND ANWENDUNGSPROZESS DERSELBEN
NAIL-HARDENING COSMETIC COMPOSITION, USE OF ALDEHYDES TO HARDEN AND STRENGTHEN NAILS, AND METHOD FOR APPLYING SAME

(30) Priorité: 30.03.2009 FR 0901522
(43) Date de publication de la demande: 08.02.2012
(73) Titulaire: Fiabila, 28130 Maintenon (FR)
(72) Inventeur: RENARD, Christine, F-28130 Maintenon (FR); BONNEVIE, Xavier, F-28300 Mainvilliers (FR)
(74) Mandataire: Le Cloirec, Claudine
(86) Numéro de dépôt international: PCT/FR2010/050579
(87) Numéro de publication internationale: WO 2010/112751

(56) Documents cités:
- EP-A1- 1 336 346
- EP-A2- 1 238 650
- WO-A1-91/07942
- WO-A1-03/005975

## Description

L'invention concerne le domaine des compositions cosmétiques pour les ongles, et plus particulièrement des compositions cosmétiques destinées à renforcer et durcir les ongles.

Il est bien connu que les ongles, qui sont composés de kératine, sont parfois fragilisés, cassants, dédoublés ou fissurés, Outre la dégradation de l'aspect esthétique de ces ongles, il en résulte des désagréments importants pour la personne dans sa vie quotidienne, principalement lorsque ces défauts concernent les ongles des mains : accrochage dans les textiles, les vêtements ou l' aggravation des fissurations lors de chaque préhension.

Jusqu'à maintenant, différentes solutions ont été apportées à ce problème, mais ces dernières sont soit imparfaites, soit génèrent d'autres inconvénients. On peut ainsi citer :
L'utilisation de formaldéhyde, dans des proportions pouvant aller jusqu'à 5 % en poids dans la composition cosmétique, est connue depuis longtemps. Cependant ce composé est maintenant très étroitement surveillé en raison de ses effets toxiques sur l'organisme, avec des effets cancérigènes avérés. Cette molécule est en outre un allergène puissant.

L'utilisation de glyoxal, un dialdéhyde, a été décrite dans le brevet GB 2 196 978 dès 1986. Ce produit est pourtant nocif par inhalation, irritant pour la peau et les yeux, et peut entraîner une sensibilisation de la peau.

L'utilisation du glutaraldéhyde est décrite dans le brevet GB 2 250 196 en association avec un sel d'aluminium soluble dans l'eau pour durcir les sabots des chevaux, composés également de kératine. Cependant, il s'avère que cet aldéhyde est toxique par inhalation et par ingestion, peut provoquer des brûlures, et peut entraîner une sensibilisation par contact avec la peau : il semble donc tout à fait inapproprié pour les ongles humains.

Le brevet GB 1 183 513 indique que l'action durcissante des aldéhydes sur les ongles décroît lorsque le nombre d'atomes de carbone augmente. Il préconise l'utilisation de mono-aldéhydes en C₁ à C₄, plus particulièrement de l'acétaldéhyde ou de di-aldéhydes pour renforcer la kératine des ongles à des teneurs comprises entre 3 et 5 %.

Le citral (sous la forme d'un mélange de ses deux isomères géranial et néral), décrit dans le brevet EP1408917, est certes également reconnu pour son action de durcisseur pour l'ongle, mais peut entraîner une sensibilisation par contact avec la peau et est classé parmi les allergènes détectés (selon la liste des substances allergènes citées dans le 7^{ème} amendement de la directive européenne 76/768/CEE). Il est donc de moins en moins utilisé en cosmétique. En outre, ces molécules comportent une double liaison, ce qui rend le citral sensible aux U.V., et donc sujet au jaunissement.

Cette longue liste d'inconvénients relatifs aux aldéhydes mis en oeuvre dans des compositions cosmétiques pour les ongles a incité l'homme du métier à rechercher d'autres types de molécules non aldéhydiques pour durcir et renforcer la solidité des ongles humains.

Parmi les autres durcisseurs des ongles, le document US 4,919,920 décrit l'incorporation d'iode ou de fluer sous forme d'ions dans une composition cosmétique aqueuse sans solvant organique, éventuellement en présence d'un polyol tel que la glycérine. Un tel usage n'est pas approprié aux vernis à ongles qui comprennent une phase importante de solvants organiques. L'iode peut aussi provoquer des allergies chez certains utilisateurs.

Il subsiste donc un réel besoin de trouver un composé durcisseur des ongles qui palie les inconvénients précités et qui puisse être mis en oeuvre dans des compositions cosmétiques variées, c'est à dire de natures différentes, à savoir vernis, gels ou huiles de soin par exemple.

Un premier but de l'invention est donc de proposer la mise en oeuvre et l'utilisation d'une famille de composés destinés à renforcer la structure de la kératine de l'ongle en la durcissant, ne présentant pas les inconvénients mentionnés ci-dessus.

Un autre but de l'invention est de proposer des composés qui ne sont pas sensibles au vieillissement, et notamment au jaunissement.

Encore un but de l'invention est de proposer un composé durcisseur pour les ongles ne présentant pas une odeur désagréable pour l'utilisateur, à des concentrations inférieures à quelques pourcents en poids.

A cet effet, la présente invention propose l'utilisation, en tant qu'agent durcisseur pour les ongles, de mono-aldéhyde de formule (I) : R-CHO, R étant un radical alkyle linéaire en C₅-C₁₂, ou un alcényle en C₅-C₁₂ à double liaison non conjuguée avec le groupement aldéhyde -CHO, de préférence n'appartenant pas à la liste des substances allergènes citées dans le 7^{ème} amendement de la directive européenne 76/768/CEE.

Il a, en effet, été constaté de manière surprenante et contrairement aux indications de l'art antérieur (en particulier du brevet GB 1 183 513) que des mono-aldéhydes présentant des radicaux alkyles ou alcényles comportant de 5 à 12 atomes de carbone présentent des propriétés durcissantes pour les ongles.

En outre, ces aldéhydes ne présentent pas de jaunissement dans le temps et ne présentent pas d'odeur désagréable pour l'utilisateur à des concentrations inférieures ou égales à 1 % en poids. Ainsi, l'aldéhyde de formule (I) peut être utilisé dans une composition cosmétique pour les ongles.

De manière avantageuse, ladite composition cosmétique renferme une concentration en aldéhyde de formule (I) inférieure ou égale à 1 % en poids, de préférence inférieure ou égale à 0,5 % en poids. De manière encore avantageuse, la composition cosmétique renferme une concentration en aldéhyde de formule (I) inférieure ou égale à 0,1 % en poids, de préférence inférieure ou égale à 0,05 % en poids.

Il a été également constaté que les propriétés durcissantes sur les ongles sont présentes, même à des concentrations inférieures ou égales à 0,01 % en poids en aldéhyde de formule (I).

L'aldéhyde de formule (I) peut avantageusement être choisi parmi l'hexanal, l'heptanal, l'octanal, le nonanal, le dodécanal, le trans-4 décénal, l'undécanal et l'aldéhyde undécylénique, ou un mélange de ceux-ci.

Ces aldéhydes ont l'avantage de ne pas présenter de danger pour la santé (sensibilisation, allergie, toxicité, ni effet cancérigène, au contraire de certains actifs de l'art antérieur).

Par exemple, de manière surprenante, l'hexanal, qui est utilisé dans le domaine de l'agroalimentaire notamment pour parfumer des boissons, s'est révélé être actif comme durcisseur pour les ongles. L'hexanal, qui peut être issu de traitements enzymatiques de l'huile de tournesol, est déjà connu comme substance aromatisante naturelle.

Selon la présente invention, l'aldéhyde de formule (I) peut être incorporé dans une composition cosmétique qui peut être une composition filmogène, tel qu'un vernis à ongles coloré ou incolore, une sous-couche de vernis ou une vernis pelable, ou peut être sous la forme d'une émulsion ou d'une solution aqueuse, d'une huile de soin, ou d'un gel. Les compositions cosmétiques filmogène, telles que les vernis, sont préférées car elles peuvent rester durablement sur l'ongle.

La présente invention concerne également toute composition de vernis à ongles, renfermant une concentration inférieure ou égale à 1 % en poids d'au moins un mono-aldéhyde de formule (I) en tant qu'agent durcisseur pour les ongles, et se présentant de préférence sous la forme d'un vernis à ongle coloré ou incolore, d'une sous-couche de vernis ou d'un vernis pelable.

Les études d'efficacité desdites compositions montrent que ces aldéhydes sont actifs à plusieurs niveaux sur les défauts des ongles : ongles fissurés, ongles fins, ongles cassants, ongles dédoublés, dureté de l'ongle, fragilité de l'ongle, sans provoquer de réactions allergiques.

La présente invention concerne également un procédé pour durcir les ongles, notamment des ongles fissurés, fins, cassants, mous ou dédoublés, consistant à appliquer de manière topique sur lesdits ongles et/ou leur pourtour, un mono-aldéhyde de formule (I) : R-CHO, R étant un radical alkyle linéaire en C₅-C₁₂, ou un alcényle en C₅-C₁₂ à double liaison non conjuguée avec le groupement aldéhyde -CHO. L'aldéhyde de formule (I) est avantageusement présent dans une composition cosmétique pour les ongles, cette composition cosmétique pouvant se présenter notamment sous la forme d'un vernis à ongle coloré ou incolore, d'une sous-couche de vernis, d'un vernis pelable, d'une huile de soin, d'une émulsion ou solution aqueuse, ou d'un gel,

Les exemples suivants permettent d'illustrer, de manière non limitative, la présente invention. Dans l'ensemble des formulations, la concentration des différents constituants est exprimée en % en poids du poids total de la composition cosmétique.

### Exemple 1

Une composition cosmétique sous la forme d'un vernis thixotrope a été préparée à partir des constituants suivants :

| | |
|---|---|
| Acétate de butyle | 41,00 |
| Acétate d'éthyle | 20,60 |
| Nitrocellulose | 14,00 |
| Résine polyester | 9,50 |
| Acétyltributylcitrate | 6,00 |
| Alcool isopropylique | 6,00 |
| Stéaralkonium bentonite | 1,30 |
| Résine styrène/acrylique | 1,30 |
| Benzophénone-1 | 0,20 |
| Polyvinylbutyral | 0,05 |
| Hexanal | 0,05 |
| | 100,00 |

Cette composition se présente ici sous la forme d'un vernis, incolore et transparent après application sur l'ongle présentant une teneur en extrait sec égal à 31,3 % en poids (mesuré à 100 °C). Une ou plusieurs matières colorantes choisies parmi les pigments, les colorants solubles et les particules décoratives, telles que les nacres et les paillettes, peuvent être ajoutées.

### Caractéristiques physiques :

Ont été testées :
- La brillance : 87 (sur une échelle de 100). Elle est mesurée avec un brillancemètre Minolta 268 (angle d'incidence 60°) pour une application faite sur carte type LENETA.
- La dureté, mesurée à l'aide d'un pendule de "Persoz" sur le film sec formé par l'application d'une couche de 100 µm d'épaisseur de la composition ci-dessus sur une plaque de verre, et séché une nuit à température ambiante (20 °C), la valeur obtenue est de 210 secondes.
- Bonne adhérence sur verre avec une valeur comprise entre 0 à 1 (sur une échelle de 5). Le film de vernis formé sur verre est griffé en quadrillage avec un peigne de six lames type SHEEN 750/1. Un ruban adhésif appliqué sur les griffures est arraché, et l'on observe que moins de 5 % de la surface du film est arrachée.
- Les viscosités, mesurées au viscosimètre Brookfield LVT avec l'aiguille n° 3 à 25 °C à 6 tours/min et à 60 tours/min pendant une minute, présentaient les valeurs suivantes : (en mPa.s, respectivement à 6 Tr/min - 60 Tr/min - 6 Tr/min) 2400 - 960 - 1350. Ce qui correspond à une bonne aptitude à l'étalement du vernis.

L'hexanal, à cette concentration, ne présente pas une odeur désagréable pour l'utilisateur.

### Tests in vivo :

La composition de l'exemple 1 a fait l'objet de tests in vivo sur un panel de 22 personnes âgées d'au moins 30 ans pendant quatre semaines pour en évaluer l'efficacité et la tolérance. Aucune des personnes testées ne présentait de lésion au niveau des zones cibles (ici le pourtour des ongles des mains), ni d'affection dermatologique, Le vernis a été appliqué dans des conditions normales d'emploi (à savoir trois applications par semaine). Ces résultats sont présentés dans le tableau ci-dessous :

**Tableau 1**

| Nombre de sujets ayant constaté une amélioration | | | | | |
|---|---|---|---|---|---|
| (nombre de sujets testés : 22) | | | | | |
| Amélioration | Légère | Modérée | Nette | Très nette | Total |
| Fragilité de l'onqle | 6 | 7 | 2 | 3 | 18/22 = 82 % |
| Ongle fissuré | 7 | 1 | 0 | 1 | 9/14 = 64 % |
| Ongle fin | 8 | 2 | 4 | 2 | 16/22 = 73 % |
| Relief de la tablette | 7 | 1 | 3 | 0 | 11/22 = 50 % |
| Ongle cassant | 5 | 3 | 4 | 3 | 15/22 = 68 % |
| Ongle dédoublé | 5 | 4 | 3 | 2 | 14/20 = 70 % |
| Dureté de l'ongle | 3 | 5 | 5 | 5 | 18/22 = 82 % |
| Amélioration globale de la qualité de l'ongle | 3 | 3 | 9 | 5 | 20/22 = 91 % |
| Tolérance au produit | Bonne pour tous les sujets | | | | |

La tolérance a été évaluée 30 minutes après l'application et à l'issue du test de quatre semaines. Aucun signe clinique d'intolérance n'a été constaté, ni ressenti par les personnes testées. Aucun symptôme d'allergie n'a été relevé.

En outre, plus de 90 % des sujets ont trouvé une amélioration globale de la qualité de leurs ongles. Ces résultats démontrent les propriétés in vivo de durcisseur de l'ongle de l'hexanal, et ceci à une faible concentration (0,05 % en poids).

Par ailleurs, les qualités cosmétiques globales de la composition ont reçu une appréciation très favorable (notes moyennes comprises entre 16 et 17/20).

### Exemple 2

### Tests in vivo/ comparaison avec un di-aldéhyde de l'art antérieur (citral décrit dans la demande de brevet EP 1 408 917 citée en introduction) :

La composition de l'exemple 1 ainsi qu'une composition 1bis identique à celle de l'exemple 1, mais dans laquelle l'hexanal a été remplacé par du citral, dans les mêmes concentrations, ont fait l'objet de tests in vivo sur un nouveau panel de 22 personnes dans les mêmes conditions que celles des tests de l'exemple 1. Chaque personne a appliqué la composition avec hexanal sur les ongles d'une main, et la composition avec citral sur les ongles de l'autre main. Le choix main droite /main gauche était aléatoire.

Les résultats des tests comparatifs montrent une supériorité entre 8 et 20% hexanal par rapport au citral sur le plan de l'amélioration de ;
- la fragilité de l'ongle (+ 18 %)
- des ongles cassants (+10 %)
- la dureté de l'ongle (+8,5 %).

L'hexanal est donc plus apte à renforcer l'ongle, que le citral.

### Exemple 3

Une composition cosmétique sous la forme d'un vernis thixotrope identique à la composition de l'exemple 1 en remplaçant l'hexanal par le dodécanal.

Les résultats des tests in vivo réalisés sur un panel de 21 personnes dans les mêmes conditions que les tests de l'exemple 1 ont montré que plus de la moitié des sujets ont constaté une amélioration globale de l'état de leurs ongles, notamment en ce qui concerne la fragilité de l'ongle, ongle fin, ongle cassant, et dureté de l'ongle, Les notes de satisfaction générale de la composition cosmétique oscillaient entre 16,9 et 18,3/20.

### Exemple 4

Une composition cosmétique sous la forme d'un vernis thixotrope identique à la composition de l'exemple 1 en remplaçant l'hexanal par l'aldéhyde undécylénique (10-undécénal).

Les résultats des tests in vivo réalisés sur un panel de 21 personnes dans les mêmes conditions que les tests de l'exemple 1 ont montré que plus de la moitié des sujets ont constaté une amélioration globale de l'état de leurs ongles, notamment en ce qui concerne la fragilité de l'ongle, ongle fin, ongle dédoublé, et dureté de l'ongle. Les notes de satisfaction générale de la composition cosmétique oscillaient entre 16 et 17,3/20.

### Exemple 5

La composition filmogène transparente colorée dans laquelle l'hexanal est dosé à 0,20 %, comprend les constituants suivants :

| | |
|---|---|
| Acétate d'éthyle | 40,00 |
| Acétate de butyle | 26,5796 |
| Nitrocellulose | 14,00 |
| Résine polyester | 7,00 |
| Acétyltributylcitrate | 6,20 |
| Alcool isopropylique | 6,00 |
| Hexanal | 0,20 |
| Polyvinylbutyral | 0,02 |
| Red 17 | 0,0002 |
| Violet 2 | 0,0002 |

### Exemple 6

L'hexanal est incorporé dans une huile de soin destinée à renforcer l'ongle, de formulation suivante :

| | |
|---|---|
| Huile d'amande douce | 98,99 |
| Parfum | 1,00 |
| Hexanal | 0,01 |

### Exemple 7

Dans cet exemple, l'hexanal est incorporé à un gel de soin, également à une concentration de 0,01 % en poids.

| | |
|---|---|
| Eau | 83,59 |
| Glycérine | 5,00 |
| Propylène glycol | 5,00 |
| Phosphate trisodique | 3,80 |
| Carbomer | 0,90 |
| Laureth 4 | 0,80 |
| Phenonip® | 0,70 |
| Parfum | 0,20 |
| Hexanal | 0,01 |

Le Phenonip® est un produit conservateur, marque déposée, de la Société Nipa contenant un mélange de paraben en solution dans le phénoxyéthanol.

### Exemple 8

L'hexanal peut aussi être efficace dans un vernis de soin en base aqueuse, constitué de :

| | |
|---|---|
| Eau | 54,80 |
| Acétate de polyvinyl | 40,00 |
| PPG 3 méthyl éther | 3,00 |
| Citrate d'ammonium | 0,60 |
| Oxyde de Titane | 0,55 |
| Phenonip® | 0,50 |
| PEG 12 Diméthicone | 0,30 |
| Copolymère Styrène Acrylique | 0,15 |
| DC Red 30 | 0,05 |
| Hexanal | 0,05 |

Ce vernis accroche faiblement à l'ongle et est détaché de l'ongle par pelage (vernis pelable). Il peut être utilisé en tant que soin pendant la nuit.

Les différents exemples ci-dessus ont montré que l'hexanal peut être incorporé dans des compositions cosmétiques de natures différentes [toutes les compositions cosmétiques ci-dessus incluant l'hexanal à de faibles concentrations, inférieures ou égales ici à 0,05 % en poids] et qu'il présente un effet de renforcement et de durcissement des ongles.

### Exemple 9

### Tests de vieillissement et de jaunissement

Différents aldéhydes aliphatiques ont été incorporés dans une composition cosmétique, de formulation correspondant à l'exemple 2, à des concentrations respectivement de 0,001 %, 0,002 %, 0,005 % et 0,01 %.

Il s'agit du méthylbutyraldéhyde (à titre de comparaison), de l'hexanal, du nonanal, du trans 4-décénal, de l'undécanal, de l'aldéhyde undécylénique.

Le vieillissement, et en particulier la tendance au jaunissement, de chaque composition ont été testés. Les divers tests effectués comprennent un maintien d'un mois à l'étuve à 45 °C et quatre mois à l'étuve à 50 °C, ainsi qu'une exposition au "SUN TEST CPS+" c'est-à-dire dans une enceinte fermée, régulée à 50 °C sous une lampe U.V./visible de 500 Watt/m² pendant 3 x 10 heures.

Le jaunissement a été évalué par rapport à des compositions témoins des formulations identiques, conservées à température ambiante (20-25 °C) à l'abri de la lumière, pendant la même durée.

Tous les vernis testés sont restés fluides dans le temps et s'étalaient aisément et de la même façon que les témoins.

Les compositions renfermant des concentrations de 0,01 % en méthylbutyraldéhyde ou en aldéhyde undécylènique ont présenté un jaunissement après un mois d'étuve à 45 °C.

L'hexanal, le nonanal, le trans 4-décénal et l'undécanal n'ont pas présenté de jaunissement aux mêmes concentrations (0,01 % en poids).

## Revendications

1. Utilisation, en tant qu'agent durcisseur pour les ongles, de mono-aldéhyde de formule (I) : R-CHO, R étant un radical alkyle linéaire en C₅-C₁₂, ou un alcényle en C₅-C₁₂ à double liaison non conjuguée avec le groupement aldéhyde -CHO,

2. Utilisation selon la revendication 1 **caractérisée en ce que** l'aldéhyde de formule (I) est présent dans une composition cosmétique pour les ongles.

3. Utilisation selon la revendication 2, **caractérisée en ce que** la composition cosmétique renferme une concentration en aldéhyde de formule (I) inférieure ou égale à 1 % en poids, de préférence inférieure ou égale à 0,5 % en poids,

4. Utilisation selon la revendication ou 2, **caractérisée en ce que** sa composition cosmétique renferme une concentration en aldéhyde de formule (I) inférieure ou égale à 0,1 % en poids, de préférence inférieure ou égale à 0,05 % en poids.

5. Utilisation selon les revendications 2 à 4, **caractérisée en ce que** la composition renferme une concentration en aldéhyde de formule (I) inférieure ou égale à 0,01 % en poids.

6. Utilisation selon les revendications 2 à 5, **caractérisée en ce que** l'aldéhyde de formule (I) est choisi parmi l'hexanal, l'heptanal, l'octanal, le nonanal, le dodécanal, le trans-4 décénal, l'undécanal et l'aldéhyde undécylénique, ou un mélange de ceux-ci.

7. Utilisation selon les revendications 2 à 6, **caractérisée en ce que** la composition cosmétique est une composition filmogène, tel qu'un vernis à ongle coloré ou incolore, une sous-couche de vernis, ou un vernis pelable.

8. Utilisation selon les revendications 2 à 6, **caractérisée en ce que** la composition cosmétique pour les ongles est sous la forme d'une émulsion ou d'une solution aqueuse, d'une huile de soin, ou d'un gel,

9. Composition de vernis à ongles **caractérisée en ce qu'**elle renferme une concentration inférieure ou égale à 1 % en poids d'au moins un mono-aldéhyde de formule (I) : R-CHO, R étant un radical alkyle linéaire en C₅-C_{12,} ou un alcényle en C₅-C₁₂ à double liaison non conjuguée avec le groupement aldéhyde-CHO, en tant qu'agent durcisseur pour les ongles,

10. Composition de vernis à ongles selon la revendication 9, **caractérisée en ce qu'**elle se présente sous la forme d'un vernis à ongle coloré ou incolore, d'une sous-couche de vernis ou d'un vernis pelable.

11. Procédé pour durcir les ongles, notamment des ongles fissurés, fins, cassants, mous ou dédoublés, consistant à appliquer de manière topique sur lesdits ongles et/ou leur pourtour, un mono-aldéhyde de formule (I) : R-CHO, R étant un radical alkyle linéaire en C₅-C₁₂, ou un alcényle en C₅-C₁₂ à double liaison non conjuguée avec le groupement aldéhyde -CHO.

12. Procédé selon la revendication 11, **caractérisé en ce que** l'aldéhyde de formule (1) est présent dans une composition cosmétique pour les ongles.

13. Procédé selon la revendication 12, **caractérisé en ce que** la composition cosmétique se présente sous la forme d'un vernis à ongle coloré ou incolore, d'une sous-couche de vernis, d'un vernis pelable, d'une huile de soin, d'une émulsion ou solution aqueuse, ou d'un gel.

## Patentansprüche

1. Verwendung von Monoaldehyd der Formel (I) R-CHO als Nagelhärter, wobei R ein lineares C₅-C₁₂-Alkylradikal oder ein C₅-C₁₂-Alkenyl mit nicht konjugierter Doppelbindung mit der Aldehydgruppe -CHO ist.

2. Verwendung nach Anspruch 1, **dadurch gekennzeichnet, dass** das Aldehyd der Formel (I) in einer kosmetischen Zusammensetzung für die Nägel vorhanden ist.

3. Verwendung nach Anspruch 2, **dadurch gekennzeichnet, dass** die kosmetische Zusammensetzung eine Konzentration des Aldehyds der Formel (I) unter oder gleich 1 Gew.-%, vorzugsweise unter oder gleich 0,5 Gew.-%, einschließt.

4. Verwendung nach Anspruch 1 oder 2, **dadurch gekennzeichnet, dass** die kosmetische Zusammensetzung eine Konzentration des Aldehyds der Formel (I) unter oder gleich 0,1 Gew.-%, vorzugsweise unter oder gleich 0,05 Gew.-%, einschließt.

5. Verwendung nach den Ansprüchen 2 bis 4, **dadurch gekennzeichnet, dass** die Zusammensetzung eine Konzentration des Aldehyds der Formel (I) unter oder gleich 0,01 Gew.-% einschließt.

6. Verwendung nach den Ansprüchen 2 bis 5, **dadurch gekennzeichnet, dass** das Aldehyd der Formel (I) aus dem Hexanal, dem Heptanal, dem Octanal, dem Nonanal, dem Dodecanal, dem Trans-4-decenal, dem Undecanal und dem Undecylenaldehyd oder einem Gemisch derselben ausgewählt ist.

7. Verwendung nach den Ansprüchen 2 bis 6, **dadurch gekennzeichnet, dass** die kosmetische Zusammensetzung eine filmbildende Zusammensetzung wie ein farbiger oder farbloser Nagellack, eine Lackunterschicht oder ein abziehbarer Lack ist.

8. Verwendung nach den Ansprüchen 2 bis 6, **dadurch gekennzeichnet, dass** die kosmetische Zusammensetzung für die Nägel in Form einer Emulsion oder eine wässrigen Lösung, eines Pflegeöls oder eines Gels ist.

9. Nagellackzusammensetzung, **dadurch gekennzeichnet, dass** sie eine Konzentration unter oder gleich 1 Gew.-% mindestens eines Monoaldehyds der Formel (I) R-CHO einschließt, wobei R ein lineares C₅-C₁₂-Alkylradikal oder ein C₅-C₁₂-Alkenyl mit nicht konjugierter Doppelbindung mit der Aldehydgruppe -CHO als Härter für die Nägel ist.

10. Nagellackzusammensetzung nach Anspruch 9, **dadurch gekennzeichnet, dass** sie sich in Form eines farbigen oder farblosen Nagellacks, einer Lackunterschicht oder eines abziehbaren Lacks darstellt.

11. Verfahren zum Härten der Nägel, insbesondere von rissigen, dünnen, bruchempfindlichen, weichen oder gespaltenen Nägeln, das darin besteht, ein Monoaldehyd der Formel (I) R-CHO, wobei R ein lineares C₅-C₁₂-Alkylradikal oder ein C₅-C₁₂-Alkenyl mit nicht konjugierter Doppelbindung mit der Aldehydgruppe -CHO ist, topisch auf die Nägel und/oder ihre Kontur aufzutragen.

12. Verfahren nach Anspruch 11, **dadurch gekennzeichnet, dass** das Aldehyd der Formel (I) in einer kosmetischen Zusammensetzung für die Nägel vorhanden ist.

13. Verfahren nach Anspruch 12, **dadurch gekennzeichnet, dass** sich die kosmetische Zusammensetzung in Form eines farbigen oder farblosen Nagellacks, einer Lackunterschicht, eines abziehbaren Lacks, eines Pflegeöls, einer Emulsion oder wässrigen Lösung oder eines Gels darstellt.

## Claims

1. The use, as a hardening agent for nails, of a mono-aldehyde of formula (I): R-CHO, R being a linear C₅-C₁₂ alkyl radical, or C₅-C₁₂ alkenyl with a double bond, non-conjugate with the aldehyde group -CHO.

2. The use according to claim 1, **characterized in that** the aldehyde of formula (I) is present in a cosmetic composition for nails.

3. The use according to claim 2, **characterized in that** the cosmetic composition contains a concentration of an aldehyde of formula (I) of less than or equal to 1% by weight, preferably less than or equal to 0.5% by weight.

4. The use according to claim 1 or 2, **characterized in that** the cosmetic composition contains a concentration of an aldehyde of formula (I) of less than or equal to 0.1% by weight, preferably less than or equal to 0.05% by weight.

5. The use according to claims 2 to 4, **characterized in that** the composition contains a concentration of an aldehyde of formula (I) of less than or equal to 0.01% by weight.

6. The use according to claims 2 to 5, **characterized in that** the aldehyde of formula (I) is selected from hexanal, heptanal, octanal, nonanal, dodecanal, trans-4-decenal, undecanal and undecylenic aldehyde, or a mixture thereof.

7. The use according to claims 2 to 6, **characterized in that** the cosmetic composition is a film-forming composition, such as a colored or colorless nail varnish, a varnish sublayer, or a peelable varnish.

8. The use according to claims 2 to 6, **characterized in that** the cosmetic composition for nails is in the form of an emulsion or an aqueous solution, an oil care, or a gel.

9. A nail varnish composition **characterized in that** it contains a concentration of less than or equal to 1% by weight of at least one mono-aldehyde of formula (I): R-CHO, R being a linear C₅-Cₗ₂ alkyl radical, or C₅-C₁₂ alkenyl with a double bond, non-conjugate with the aldehyde group -CHO, as a hardening agent for nails.

10. The nail varnish composition according to claim 9, **characterized in that** it appears in the form of a colored or colorless nail varnish, a varnish sublayer or a peelable varnish.

11. A method for hardening nails, notably cracked, thin, brittle, soft or split nails, consisting of topically applying on said nails and/or their perimeter, a mono-aldehyde of formula (I): R-CHO, R being a linear C₅-C₁₂ alkyl radical, or C₅-C₁₂ alkenyl with a double bond, non-conjugate with the aldehyde group -CHO.

12. The method according to claim 11, **characterized in that** the aldehyde of formula (I) is present in a cosmetic composition for nails.

13. The method according to claim 12, **characterized in that** the cosmetic composition appears in the form of a colored or colorless nail varnish, a varnish sublayer, a peelable varnish, and oil care, and emulsion or aqueous solution, or a gel.
